# EUROPÄISCHE PATENTANMELDUNG

(11) **EP 0 759 330 A2**
(43) Veröffentlichungstag der Anmeldung: **26.02.1997**
(21) Anmeldenummer: 96110552.5
(22) Anmeldetag: 29.06.1996
(51) Int. Cl.: B08B 9/46, G01N 1/26, G01N 1/00

(54) **Vorrichtung zur Schaffung einer Fluidverbindung zwischen einem festen Fluidanschluss und einem bewegten Behälter**

(30) Priorität: 23.08.1995 CH 2402/95
(71) Anmelder: ELPATRONIC AG, CH-6303 Zug (CH)
(72) Erfinder: Gysi, Peter, 5454 Bellikon (CH)

(57) **Zusammenfassung**

Oberhalb der auf einer Fördereinrichtung geförderten Behälter (5,5',5'',5''') ist die Vorrichtung (1) mit einem umlaufenden Band (6) versehen, welches Durchbrechungen (10-17) aufweist. Diese stehen über Kanäle (23,24,25) in Verbindung mit feststehenden Fluidanschlüssen (19,20,21) und begleiten durch entsprechenden Antrieb des Bandes (6) die Behälter entlang des Förderweges. Dadurch wird auf einfache Weise eine Verbindung zwischen den bewegten Behältern und den feststehenden Fluidanschlüssen geschaffen, welche auch für hohe Fördergeschwindigkeiten der Behälter geeignet ist.

## Beschreibung

Die Erfindung betrifft eine Vorrichtung zur Schaffung einer Fluidverbindung zwischen mindestens einem festen Fluidanschluss und mindestens einem von einer Mehrzahl auf einer Förderanlage geförderten Behältern.

Eine solche Vorrichtung ist zur Entnahme eines Fluids bei der Inspektion von Flaschen aus EP-A-0 579 952 bekannt. Dabei wird eine Gasprobe aus den Flaschen entnommen und an einen festen Anschluss abgegeben, an dem ein Analysegerät für die Gasprobe vorgesehen ist. Die Flaschen werden auf einer Karussellförderanlage gefördert, was aufwendig ist. Eine Vorrichtung zur Einbringung eines Flüssigkeitsstahls in eine Flasche zu Inspektionszwecken ist ferner aus DE-U-94 03 641 bekannt. Dort wird oberhalb der linear geförderten Behälter ein Einspritzrad angeordnet, welches sich über der Förderstrecke dreht. Dies ergibt gegenüber dem Karussell eine konstruktive Vereinfachung, doch ist die Zeitdauer der Verbindung, also diejenige Zeitdauer, in welcher die Flüssigkeit in die Flasche eingebracht werden kann, sehr kurz, insbesonders bei hohen Behälterförderleistungen.

Der Erfindung liegt deshalb die Aufgabe zugrunde, eine Vorrichtung bereitzustellen, zur Schaffung einer Fluidverbindung zwischen einem geförderten Behälter und einem festen Fluidanschluss, also z.B. einem festen Anschluss zur Zufuhr von aus dem Behälter entnommenem Gas zu einer Analyseeinrichtung oder einem festen Anschluss für ein Fluid, das in den Behälter eingebracht werden soll. Dabei soll die Vorrichtung konstruktiv einfach und auch für hohe Behälterförderraten geeignet sein.

Diese Aufgabe wird bei einer Fördereinrichtung der eingangs genannten Art dadurch gelöst, dass ein angetriebenes, im wesentlichen bandförmiges Element vorgesehen ist, dessen eine Seite mit dem Fluidanschluss in Verbindung steht, und dessen andere Seite der Behälteröffnung zugewandt ist, und welches mindestens einen Durchbruch aufweist, der eine Verbindung zwischen Fluidanschluss und Behälter bildet.

Dadurch, dass ein bandförmiges Element vorgesehen ist, das angetrieben den geförderten Behältern folgen kann, ergibt sich sowohl die Möglichkeit einer einfachen Konstruktion als auch die Möglichkeit eines zeitlich langen Kontaktes zwischen Band und Behälter, da die Bandlänge entsprechend gewählt werden kann.

Das bandförmige Element kann von einer Rolle abgewickelt werden und nach einmaligen Gebrauch entsorgt werden; dies vor allem bei der Verwendung der Vorrichtung zur Behandlung stark kontaminierter oder stark kontaminationsgefährdeter Behälter, z.B. in der chemischen Industrie oder pharmazeutischen Industrie. Bei einer speziellen Ausführungsform ist das bandförmige Element aber ein Endlosband, welches umläuft und nach der Herstellung der Verbindung mit einem Behälter wieder zur Herstellung der Verbindung mit einem weiteren Behälter zurückläuft.

Bei einer Ausführungsform läuft das Element auf einem Führungselement, welches bevorzugterweise zugleich die Fluidzufuhr zu dem oder die Fluidwegfuhr von dem Element sicherstellt. Insbesondere bei einer geringen Länge des Elementes kann aber auf das Führungselement auch verzichtet werden.

Im folgenden werden Ausführungsbeispiele der Erfindung anhand der Zeichnungen näher erläutert. Dabei zeigt
Figur 1 perspektivisch eine Reihe geförderter Flaschen zusammen mit einer Ausführungsform der Vorrichtung;
Figur 2 eine teilweise geschnittene Seitenansicht der Vorrichtung von Figur 1;
Figur 3 eine behälterseitige Ansicht eines Führungselementes;
Figur 4 eine behälterseitige Ansicht eines weiteren Führungselementes;
Figur 5 eine teilweise geschnittene Teilseitenansicht einer weiteren Ausführungsform der Vorrichtung;
Figur 6 eine Schnittansicht der Vorrichtung von Figur 5 in Förderrichtung; und
Figur 7 eine teilweise geschnittene Teilseitenansicht einer weiteren Ausführungsform der Vorrichtung.

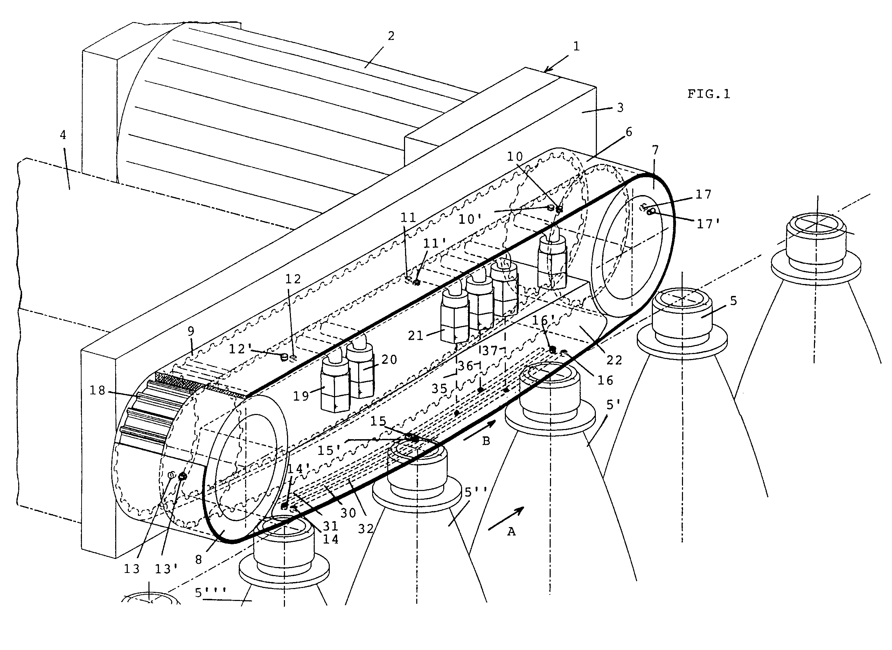

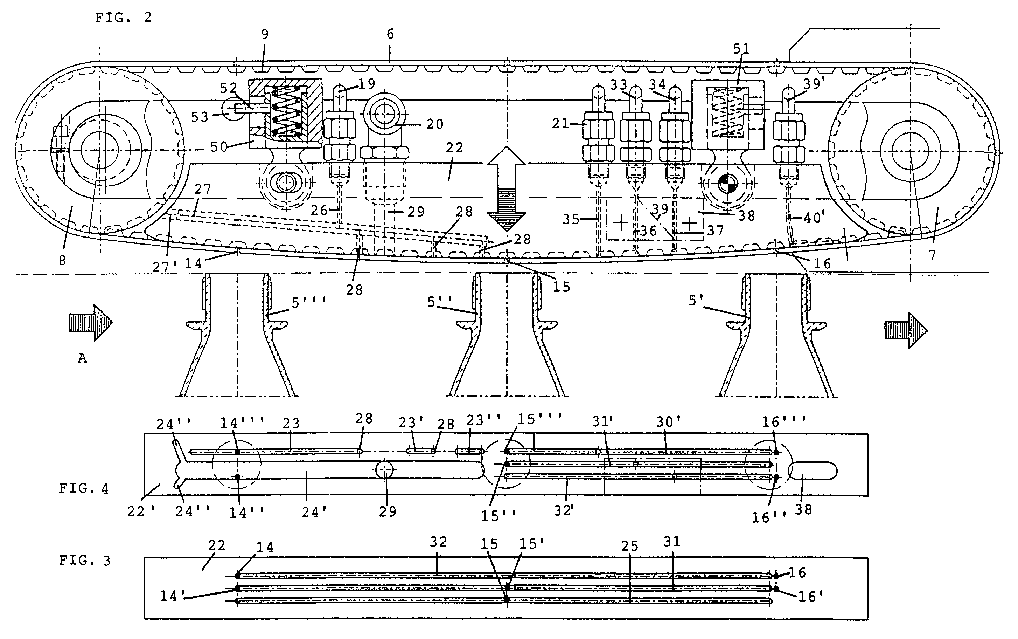

Figur 1 zeigt in schematischer, perspektivischer Ansicht eine erste Ausführungsform der Vorrichtung 1. Dabei ist eine Tragplatte 3 vorgesehen, an welcher ein Antriebsmotor 2 und ein Anschlusskasten 4 für die Fluidanschlüsse und allenfalls zur Aufnahme von Analyseeinrichtungen vorgesehen ist. An der Vorderseite der Platte 3 ist das angetriebene bandförmige Element 6 vorgesehen, welches im gezeigten Beispiel ein Endlosband ist, das mittels zweier Umlenkrollen 7 und 8 umgelenkt wird. Die Rolle 7 wird dabei durch den Elektromotor 2 angetrieben, so dass die Geschwindigkeit des Bandes zwischen den Umlenkrollen im wesentlichen gleich der in Pfeilrichtung A geförderten, nur teilweise dargestellten Behälter bzw. Flaschen 5, 5', 5'', 5''' usw. eingestellt werden kann. Der Antrieb des Bandes kann z.B. dadurch erfolgen, dass die eine Hälfte des Bandes als Zahnriemen mit den Zähnen 9 ausgestaltet ist, und dass die Umlenkrollen 7 und 8 auf einem Teil ihrer Breite mit entsprechenden Zähnen 18 versehen sind. Das Element 6 ist in dem gezeigten Beispiel als riemenförmiges Band, also als Band mit einem rechteckigen Querschnitt dargestellt. Der Bandquerschnitt könnte aber natürlich auch quadratisch, rund oder elliptisch sein. In dem Band sind mehrere Durchbrechungen 10, 10' bis 17, 17' angeordnet. Deren Abstand voneinander ist so gewählt, dass die Durchbrechungen jeweils im Bereich einer Flaschenmündung liegen, wie dies für die Durchbrechungen 14, 14', 15, 15' und 16, 16' ersichtlich ist, welche sich in der gezeigten Stellung über der Mündung der Flaschen 5''' bzw. 5'' bzw. 5' befinden. Da die Antriebsgeschwindigkeit des Bandes so gewählt wird, dass sich das Band zwischen den Umlenkrollen 7 und 8 mit der selben Geschwindigkeit bewegt wie die Behälter, so begleitet jeweils die entsprechende Durchbrechung im Band die ihr zugeordnete Behältermündung. Die Durchbrechung 14 und auch die Durchbrechung 14' wandert also zusammen mit dem Behälter 5''' von der Umlenkrolle 8 zur Umlenkrolle 7 und steht während dieser Zeit mit der Behältermündung in Kontakt. Damit über die jeweilige Durchbrechung eine Fluidverbindung von einem festen Anschluss zu dem Behälter geschaffen werden kann, muss auf der Innenseite des Bandes das Fluid zu der Jeweiligen Durchbrechung herangeführt werden. Im gezeigten Beispiel ist dazu ein Führungselement 22 vorgesehen, welches an seiner dem Band zugewandten Seite mit in Bandlaufrichtung verlaufenden Kanälen 30, 31 und 32 versehen ist, welche in der Form von gegen das Band hin offenen Nuten ausgeführt sind. Jeder dieser Kanäle steht durch Leitungen 35, 36, 37 im Bandführungselement 22 in Verbindung mit mindestens einem festen Fluidanschluss. In dem gezeigten Beispiel sind mehrere Fluidanschlüsse dargestellt, wobei der Fluidanschluss 21 mit dem Kanal 30 in Verbindung steht, der Fluidanschluss 33 mit dem Kanal 31 und der Fluidanschluss 34 mit dem Kanal 32 (siehe Figur 2). Diese Fluidanschlüsse sind feststehend und sind über Leitungen, die in den Anschlusskasten 4 geführt sind, mit entsprechenden Fluidquellen oder mit Analysegeräten verbunden. Betrachtet man die Vorrichtung 1 z.B. als eine Vorrichtung zur Entnahme von Gasproben aus Behältern, so kann z.B. an dem Anschluss 21 eine Druckluftquelle angeschlossen sein, so dass im Kanal 30 ein Überdruck herrscht und über die Durchbrechung 14' in dem Band 6, welche auf einer Seite mit dem Kanal 30 in Verbindung steht und auf der anderen Seite oberhalb der Behältermündung des Behälters 5''' liegt, Druckluft in den Behälter eingeblasen wird. Dies ist bei der Entnahme von Gasproben bekannt. Die Gasprobe selber wird über die Durchbrechung 14 abgesaugt, die über den Kanal 32 und die Leitung 37 mit dem Fluidanschluss 34 verbunden ist, an welchem ein das Fluid ansaugendes Analysegerät angeschlossen ist, z.B. ein Massenspektrometer. Dadurch, dass sich der Behälter mit einer Geschwindigkeit in Richtung des Pfeils A bewegt, die gleich gross ist wie die Geschwindigkeit des Bandes in Richtung B, ergibt sich, dass die Durchbrechung 14 den Behälter auf der gesamten Länge des Kanals 32 begleitet und während dieser Begleitung Gas aus dem Behälter absaugt. Dasselbe gilt natürlich auch für die anderen Behälter, die unterhalb einer der anderen Durchbrechungen 15, 15' und 16, 16' angeordnet sind, wobei die Durchbrechung 15' ebenfalls mit dem druckluftgespiesenem Kanal 30 in Verbindung steht und die Durchbrechung 15 über den Kanal 31 und die Leitung 36 mit dem feststehenden Fluidanschluss 33 in Verbindung steht. An diesem ist ein zweites Analysegerät angeschlossen. Die Durchbrechungen 16' und 16 sind wieder gleich angeordnet wie die Durchbrechungen 14' und 14, so dass hier wieder das erste Analysegerät zum Einsatz kommt. Die Prüfung der Flaschen erfolgt also in diesem Beispiel immer abwechselnd durch das erste und das zweite Analysegerät, was durch die Anordnung der Durchbrechungen bewirkt wird. Für die weiteren Behälter, die im Laufe ihrer Förderung mit den Durchbrechungen 13, 13', 12, 12', 11, 11' usw. in Verbindung treten, gilt dies ebenfalls. Die Verbindung zwischen Behälter und dem Element 6 kann dabei so erfolgen, dass die Behältermündung jeweils an dem Band anliegt. Es kann aber auch so sein, dass das Band von der Behältermündung beabstandet ist. Unter Verbindung im vorliegenden Sinn wird also nicht zwingend eine fluiddichte Verbindung verstanden, sondern lediglich, dass das Fluid aus dem Behälter zu dem feststehenden Anschluss gelangen kann.

Damit die Synchronisation zwischen den Behältern und dem Band gegeben ist, wird bei der Fördereinrichtung eine entsprechende Einrichtung vorgesehen, die den Behälterabstand konstant hält, so dass die Mündungen der Behälter jeweils genau den Abstand der Durchbrechungen voneinander einhalten. Diese Einrichtung kann z.B. eine Förderschnecke sein, welche die Behälter in dem Bereich unter der Vorrichtung 1 fördert. Die Höhe der Vorrichtung über der Fördereinrichtung für die Behälter wird einstellbar ausgeführt, damit die Vorrichtung an verschiedene Behälterhöhen angepasst werden kann. Durch die Auswechslung des Bandes 6 kann ferner eine Anpassung an verschiedene Behältermündungsabstände erfolgen, in dem ein Band mit entsprechend beabstandeten Durchbrechungen eingesetzt wird. Sofern auf das Bandführungselement 22 verzichtet wird, was insbesondere bei kurzen Abständen zwischen den Umlenkungsrollen 7 und 8 möglich ist, sind die festen Anschlüsse 21, 33 usw. so ausgebildet, dass sie direkt am Band anliegen und sich in länglicher Form in Bandlaufrichtung erstrecken, so dass die Anschlüsse selber direkt die Funktion der Kanäle 30, 31 usw. übernehmen.

Für den Fall, dass mittels der Vorrichtung 1 eine Flüssigkeit oder ein Gas in die Flaschen eingeführt wird, so erfolgt der Vorgang an sich gleich wie bereits bei der Gasprobenentnahme beschrieben. In diesem Fall wird das in die Flaschen einzuführende Fluid aus einer Fluidquelle, z.B. zu dem Anschluss 21 geführt und gelangt von diesem in den Kanal 30 und von dort über die Durchbrechungen in dem bandförmigen Element 6 in die Behälter, z.B. über die Durchbrechung 15 in den Behälter 5''. Dies erfolgt während des gesamten Weges des Behälters entlang des Kanals 30.

Wie bereits erwähnt, könnte anstelle des Endlosbandes 6 auch ein endliches Band, z.B. in der Form einer auf einer Rolle aufgerollten Kunststoffolie vorgesehen sein, welches auf grundsätzlich gleiche Weise oberhalb der Behälter mit deren Fördergeschwindigkeit abläuft und welches nach Gebrauch auf einer anderen Rolle aufgerollt wird. Eine solche Anordnung kann für bestimmte Zwecke, bei der eine hohe Kontaminationsgefahr besteht, brauchbar sein, da dabei jede Durchbrechung bzw. der entsprechende Bandabschnitt nur einmal mit einem Behälter in Kontakt oder in dessen Nähe tritt, so dass keinerlei Gefahr besteht, dass Spuren einer Substanz von dem einen Behälter zu einem anderen Behälter durch das Band übertragen werden. Natürlich sind auch die dargestellten Behälter, welche in Figur 1 Flaschen sind und z.B. rücklaufende Mehrwegflaschen sind, welche vor der Wiederbefüllung inspiziert werden, nur als Beispiel zu verstehen. Auf die gezeigte Art und Weise können grundsätzlich jede Art von Behältern mit einem feststehenden Fluidanschluss in Verbindung gebracht werden, also z.B. Kanister, Reagenzgläser, offene Ampullen, Dosen usw.

In Figur 1 ist das Band oberhalb einer linearen Förderstrecke gezeigt, was in der Regel der Fall sein wird. Das Band kann dabei ein handelsüblicher Riemen aus einem Kunststoffmaterial sein oder auch ein dünnes Stahlband, das z.B. auf einer mit Magneten besetzten Kunststoffleiste läuft. Das Band kann aber z.B. auch als Gliederband ausgeführt sein, welches eine Biegung in der Bandlängsachse erlaubt und damit eine Verfolgung eines gekrümmten Förderweges.

Figur 2 zeigt eine Seitenansicht einer Ausführungsform gemäss Figur 1, worin gleiche Bezugzeichen wie in Figur 1 gleiche Teile bezeichnen, und Figur 3 zeigt eine Draufsicht auf die Kanäle im Führungselement 22 gemäss Figur 1.

Figur 2 dient nun zusammen mit Figur 4, die eine Draufsicht auf ein Führungselement 22' zeigt, zur Erläuterung eines weiteren Ausführungsbeispiels mit den weiteren Anchlüssen 19 und 20. Ersichtlich sind hierbei zusätzlich die als Beispiel eingezeichneten Leitungen 26, 27 und 28, welche den Anschluss 19 mit einem Kanal im Führungselement 22 verbinden. Die Leitung 27 ist mit einem Stopfen 27' verschlossen. In diesem Beispiel ist der Kanal so ausgeführt, wie er in Figur 4 gezeigt ist. Dabei besteht dieser, mit dem Anschluss 19 in Verbindung stehende Kanal aus drei Abschnitten 23, 23' und 23''. Derjenige Kanal, der mit dem Anschluss 20 über die Leitung 29 verbunden ist, ist in diesem Beispiel, wie in Figur 4 ersichtlich, als breiter Kanal 24' ausgeführt, welcher so breit ist, dass er mit mehreren seitlich zueinander versetzten Durchbrechungen in Kontakt treten kann. In diesem Beispiel ist es so, dass eine Drucklufteinblasung zusätzlich über den Anschluss 19 und die Kanäle 23, 23' und 23'' erfolgt. Über den Anschluss 21 wird Druckluft in einen Kanal 30' geführt. Der Anschluss 20 steht mit einer Unterdruckquelle in Verbindung, so dass mittels des Kanals 24' Gas aus der Flasche abgesaugt werden kann. Die Kanäle 31' und 32' stehen je mit einem Analysegerät über die festen Anschlüsse 33 und 34 in Verbindung (die Zuordnung der Kanäle und Anschlüsse ist hierbei nicht identisch zum vorhergehenden Beispiel). Das Lochbild der Durchbrechungen im Band 6 ist verschieden von demjenigen im vorhergehenden Beispiel. In Figur 4 ist die Lage der Durchbrechungen 14'' und 14''', 15'' und 15''' sowie 16'' und 16''' jeweils als schwarzer Punkt angegeben. Demzufolge wird aus der Flasche 5''' über den Kanal 24' Gas abgesaugt (und im Bereich der Unterdruckquelle an die Umgebung abgegeben) und es wird über die Kanäle 23, 23' und 23''' intermittierend Druckluft eingeblasen. Wenn die Flasche 5''' bzw. die Durchbrechungen 14'' und 14''' in den Bereich der Kanäle 30', 31' und 32' gelangen, so erfolgt via Kanal 30' und Durchbrechung 14''' im stetiges Einblasen von Druckluft und via Kanal 32' und Durchbrechung 14'' ein Absaugen von Gas und Zuführen desselben zu einem ersten Analysegerät. Bei der Flasche 5'' mit dem zugeordneten Lochbild 15'', 15''' der Durchbrechungen ergibt sich für die Druckluft derselbe Ablauf, die Ansaugung von Gas hingegen erfolgt via Kanal 31' auf ein zweites Analysegerät. Bei der Flasche 5' mit den Durchbrechungen 16'' und 16''' verhält es sich gleich wie bei der Flasche 5''' und den Durchbrechungen 14'' und 14'''. Es ergibt sich damit immer ein abwechselnder Einsatz der beiden Analysegeräte. Wenn nur ein Analysegerät zum Einsatz kommen kann, z.B. bei einer Wartung des einen Gerätes, so kann, z.B. im Führungselement 22 ein auswechselbarer Block 38 vorgesehen sein, der die Leitung 36 und eine Leitung 39 anstelle von 37 aufweist (wie in Figur 2 angedeutet); auf diese Weise werden die Gasproben aller Flaschen nur dem einen am Anschluss 33 befindlichen Analysegerät zugeführt. Im Block 38 können z.B. auch Feinfilter für die Leitungen 36, 37 angeordnet sein, die auf diese Weise rasch auswechselbar sind. Den Kanal 24' weist ferner zusätzliche Seitenarme 24'' auf, welche zu Reinigungszwecken der Bandinnenseite dienen. Bei der in Figur 4 als Beispiel gezeigten Kanalanordnung des Führungselementes 22 ist noch ein Kanal 38' gezeigt, welcher mit dem Anschlussstück 39' über den Kanal 40' in Verbindung steht.

Aus diesen Beispielen ist ersichtlich, dass eine einfache Aenderung der Zuordnung zwischen den Anschlüssen und den Durchbrechungen bzw. den Behältern durch die Gestaltung und Auswechslung des die Kanäle enthaltenden Führungselementes 22 bzw. durch entsprechende Lochbilder der Durchbrechungen erfolgen kann. Beliebig viele weitere Anordnungen entsprechend den jeweiligen Aufgabenstellungen sind möglich.

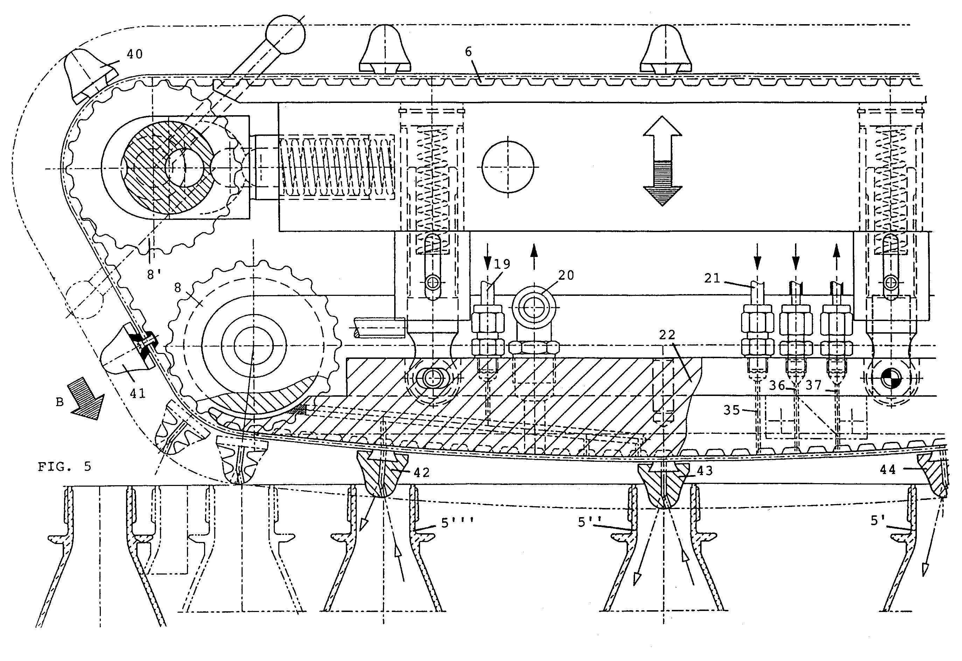

Figur 5 zeigt eine weitere Ausführungsform der Vorrichtung 1, ebenfalls mit einem endlosen Element 6, welches auch hier als Riemen und dabei teilweise als Zahnriemen ausgestaltet ist. Gleiche Bezugszeichen wie bisher bezeichnen auch hierbei gleiche Elemente. Von der Vorrichtung ist aber nur ein Teil dargestellt, d.h. nur eine der Umlenkungen. An der nicht dargestellten Umlenkungsstelle erfolgt die Umlenkung indes auf gleiche Weise wie in der Figur dargestellt. Zur Umlenkung sind in den dargestellten Beispiel 2 übereinander angeordnete Rollen 8 und 8' vorgesehen. Diese Rollenanordnung ergibt ein gutes Einlaufen und Eintauchen der in diesem Beispiel an dem Band vorgesehenen Mündungsteile 40, 41, 42, 43, 44 usw., welche jeweils in die Behälter eintauchen. Durch das Eintauchen kann die Einbringung des Fluids in den Behälter oder die Entnahme des Fluids aus dem Behälter verbessert werden. Die Mündungsteile können an dem Band auf lösbare Weise befestigt sein, so dass ein Auswechseln derselben möglich ist.

In Figur 5 sind solche Mündungsteile vorgesehen, die in den jeweiligen Behälter eintauchen können, ohne diesen zu kontaktieren. Die übrigen Funktionen, d.h. die Zuführung oder Wegfuhr des Fluids über die feststehenden Anschlüsse, die Leitungen im Führungselement 22 und über die darin angeordneten Kanäle zu den Durchbrechungen im Band bzw. zu den an den Durchbrechungen angeordneten Mündungsteilen erfolgen gleich wie bei den bereits geschilderten Beispielen.

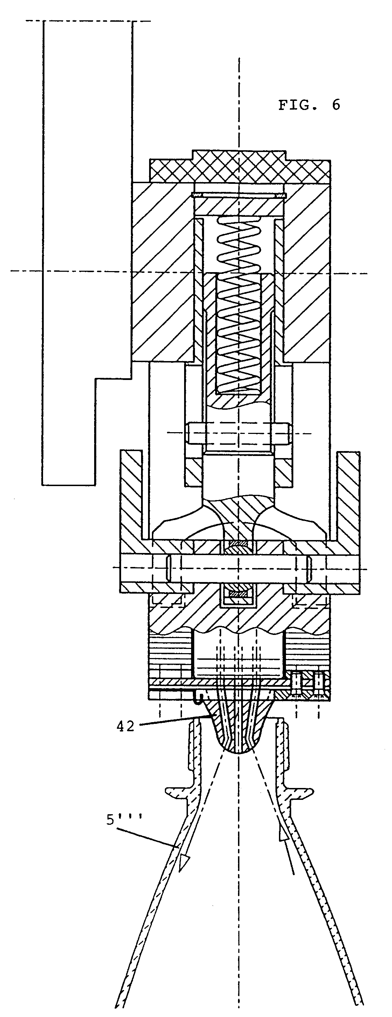

Figur 6 zeigt eine geschnittene Ansicht einer Figur 5 entsprechenden Vorrichtung in Förderrichtung. Dabei bezeichnen gleiche Bezugszeichen wie bis anhin gleiche Teile.

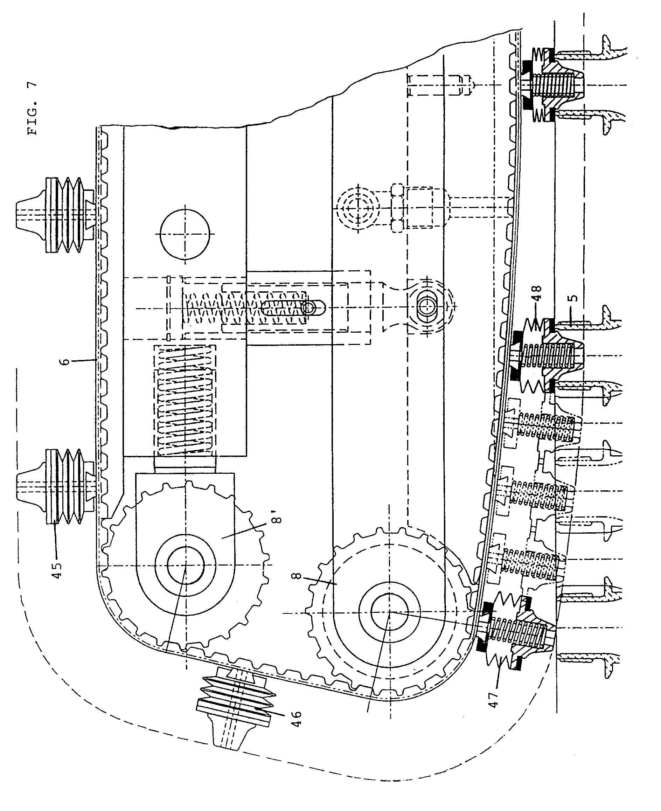

Figur 7 zeigt ein weiteres Ausführungsbeispiel der Vorrichtung, wobei auch hier, wie in dem Beispiel gemäss Figur 6, eine Umlenkung an der einen Umlenkungsstelle mit den beiden Rollen 8 und 8' erfolgt. Auch bei dieser Ausführungsform sind an dem Band sechs Mündungsteile angeordnet. Diese Mündungsteile 45, 46, 47, 48 usw. sind indes so ausgeführt, dass sich eine Kontaktnahme und ein abdichtendes Aufliegen des jeweiligen Mündungsteils an der Flaschenmündung ergibt. Dies ist für das Mündungsteil 48 dargestellt, welches an der Mündung des Behälters 5 anliegt. Die Mündungsteile sind dazu federnd ausgestaltet, so dass eine entsprechende elastische Verkürzung des Mündungsteils bei der Kontaktnahme mit der Mündung möglich ist. Mit einem dichtenden Anliegen ist z.B. zusätzlich eine Dichtigkeitskontrolle der Behälter durchführbar. Dazu wird Fluid unter Druck in den Behälter eingebracht oder Fluid aus dem Behälter entnommen, um darin Unterdruck zu erzeugen, und es wird der Druckverlauf über eine gewisse Zeit betrachtet, um ein Behälterleck festzustellen.

Die Vorrichtung 1 ist vorzugsweise höheneinstellbar über dem Förderweg angeordnet, so dass auf einfache Weise eine Anpassung an die jeweilige Behälterhöhe erfolgen kann. Wie z.B. in Figur 2 dargestellt, kann das Führungselement 22 mittels federnden Lagerungen 50, 51 befestigt sein. Dies erlaubt einerseits eine Spannung des Bandes 6 bei dessen Verlauf über das Führungselement 22. Dieses ist vorzugsweise bogenförmig ausgestaltet, um die Dichtung zwischen Bandinnenseite und den Kanälen zu verbessern. Die federnde Lagerung erlaubt weiter ein Einfedern und damit ein zerstörungsfreies Ausweichen des Führungselementes bei einer Flasche mit Deckel oder einer zu hohen Flasche. Über eine Schaltfahne 52 und einen auf diese ansprechenden Sensor 53 kann das Einfedern detektiert werden, und es kann z.B. die Vorrichtung angehalten werden oder die entsprechende Flasche kann mittels eines Ausleitsystems aus dem Förderweg entfernt werden.

## Patentansprüche

1. Vorrichtung zur Schaffung einer Fluidverbindung zwischen mindestens einem festen Fluidanschluss (19, 20, 21, 33, 34) und mindestens einem von einer Mehrzahl auf einer Förderanlage geförderten Behältern (5, 5', 5'', 5'''), dadurch gekennzeichnet, dass ein angetriebenes, im wesentlichen bandförmiges Element (6) vorgesehen ist, dessen eine Seite mit dem Fluidanschluss in Verbindung steht, und dessen andere Seite der Behälteröffnung zugewandt ist, und welches mindestens einen Durchbruch (10-17) aufweist, der eine Verbindung zwischen Fluidanschluss und Behälter bildet.

2. Vorrichtung nach Anspruch 1, dadurch gekennzeichnet, dass ein endloses bandförmiges Element (6) vorgesehen ist.

3. Vorrichtung nach Anspruch 1 oder 2, dadurch gekennzeichnet, dass das Element oberhalb des Behältermündungsbereichs mindestens abschnittsweise auf einem Führungselement (22) läuft.

4. Vorrichtung nach Anspruch 3, dadurch gekennzeichnet, dass das Führungselement mindestens einen sich in Förderrichtung erstreckenden Fluidkanal (30, 31, 32; 23, 23', 23'', 24', 24'', 30', 31', 32' ) aufweist, der mit dem mindestens einen Fluidanschluss in Verbindung steht und dessen der Förderanlage zugewandte Kanalwandung von dem bandförmigen Element gebildet wird, dessen Durchbruch im Bereich des Kanals angeordnet ist.

5. Vorrichtung nach Anspruch 3 oder 4, dadurch gekennzeichnet, dass das Führungselement im Bereich des Bandes mindestens abschnittsweise eine konvexe Krümmung aufweist.

6. Vorrichtung nach einem der Ansprüche 3 bis 5, dadurch gekennzeichnet, dass das Führungselement federnd gelagert ist.

7. Vorrichtung nach Anspruch 6, dadurch gekennzeichnet, dass eine Anordnung (52, 53) vorgesehen ist, welche bei Auslenkung des federnd gelagerten Führungselementes ein Signal abgibt.

8. Vorrichtung nach einem der Ansprüche 4 bis 7, dadurch gekennzeichnet, dass das Führungselement mindestens einen quer oder schräg zum Band verlaufenden Reinigungskanal (24'') umfasst.

9. Vorrichtung nach einem der Ansprüche 3 bis 8, dadurch gekennzeichnet, dass das Führungselement Leitungen (26, 27, 28, 29, 35, 36, 37) aufweist, die von den Fluidanschlüssen (19, 20, 21, 33, 34) zu den Kanälen führen, und dass mindestens zwei Leitungen (36, 37) in einem auswechselbaren Block (38) angeordnet sind.

10. Vorrichtung nach einem der Ansprüche 1 bis 9, dadurch gekennzeichnet, dass das Band in einem Abstand von der Oberkante der Behälter verläuft, so dass eine berührungslose Fluidverbindung geschaffen wird.

11. Vorrichtung nach einem der Ansprüche 1 bis 9, dadurch gekennzeichnet, dass an dem bandförmigen Element Mündungsteile (40-44; 45-48) vorgesehen sind, welche zum Eintauchen in die Behältermündung bestimmt sind und mindestens eine in Verbindung mit dem Durchbruch stehende Fluidleitung aufweisen.

12. Vorrichtung nach Anspruch 11, dadurch gekennzeichnet, dass die Mündungsteile (45-48) derart ausgestaltet sind, dass ein dichtendes Aufliegen auf dem Behältermündungsrand erfolgt.

13. Verwendung einer Vorrichtung nach einem der Ansprüche 1 bis 12 zur Entnahme eines Fluids aus einem Behälter, insbesonders einer zu inspizierenden Mehrweg-Flasche und/oder zur Einbringung eines Fluids in einen solchen Behälter.

14. Verwendung einer Vorrichtung nach Anspruch 12 zur Dichtigkeitsprüfung eines Behälters, insbesonders einer Mehrweg-Flasche.
